# EUROPEAN PATENT SPECIFICATION

(11) **EP 2 954 895 B1**
(45) Date of publication and mention of the grant of the patent: **21.09.2016**
(21) Application number: 15171424.3
(22) Date of filing: 10.06.2015
(51) Int. Cl.: A61K 9/20, A61K 9/24, A61K 31/192, A61K 31/194

(54) **PHARMACEUTICAL COMBINATIONS OF CONTROLLED RELEASE FLURBIPROFEN AND DIACEREIN**
PHARMAZEUTISCHE KOMBINATIONEN AUS FLURBIPROFEN MIT KONTROLLIERTER FREISETZUNG UND DIACEREIN
COMBINAISONS PHARMACEUTIQUES DE DIACÉRÉINE ET FLURBIPROFÈNE À LIBÉRATION CONTRÔLÉE

(30) Priority: 11.06.2014 TR 201406767
(43) Date of publication of application: 16.12.2015
(73) Proprietor: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); YELKEN, Gülay, 34460 Istanbul (TR)
(74) Representative: Sevinç, Erkan

(56) References cited:
- EP-A1- 2 074 990
- WO-A2-2009/040702
- WO-A2-2013/095319
- WO-A2-2013/100882

## Description

### Technical Field

The present invention relates to a pharmaceutical combination of flurbiprofen or a pharmaceutically acceptable salt thereof and diacerein or a pharmaceutically acceptable salt thereof.

Particularly, the pharmaceutical combination comprising: a controlled release layer comprising flurbiprofen or a pharmaceutically acceptable salt thereof and an immediate release layer comprising diacerein or a pharmaceutically acceptable salt thereof, and pharmaceutically acceptable excipients.

### Background of the invention

Flurbiprofen is a propionic acid derivative which is an NSAID (non-steroidal anti-inflammatory drug), having analgesic and anti-inflammatory activities. Its chemical structure is illustrated with Formula 1 given below.

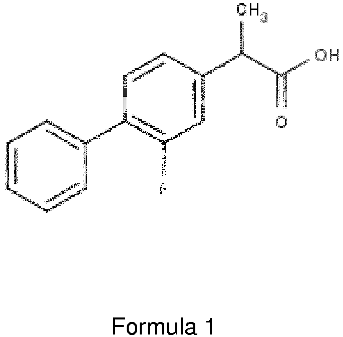

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful severe menstruation and migraine. It is in the market under the brandname of ANSAID^{®} in strength of 50, 100, 200 and 300 mg. It is recommended 2, 3 or 4 times a day dose. Multiple daily dosing leads serious side effects which decrease the patient's quality of life.

The most frequent side effects of immediate release flurbiprofen are including gastrointestinal (GI) adverse effects such as inflammation, spontaneous gastric bleeding, ulceration and perforation of the stomach, which can be life threatening. Another disadvantage of flurbiprofen is that it is practically insoluble in water. This makes it difficult to prepare controlled release formulations.

Another molecule that can be used especially in the treatment of osteoarthritis is diacerein disclosed in the patent US4244968A. It is interleukin-1 inhibitor having anthraquinone structure and chemical structure thereof is shown in Formula 2.

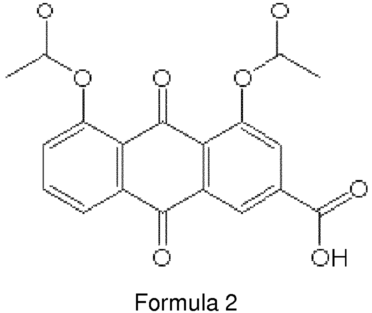

Diacerein is also available in the market under the brand names Artrodar^{®} and Rexena^{®} and it is in the form of capsule in the strength of 50 mg for once daily use. It is known that diacerein has a different mechanism of action than the other NSAIDs. It works by blocking the actions of interleukin-1 beta, a protein involved in the inflammation process

While combining more than one molecule in one dosage form is increasing the patients' quality of life, many challenges also occur such as(a) the physicochemical compatibility between the different active agents and/or between the active agents and the excipients used; and (b) the therapeutical compatibility between the two active agents regarding their pharmacokinetic and/or pharmaceutical properties in order that the posology of the combined formulation allows to obtain safe and efficient plasma levels of both pharmacological agents.

Use of flurbiprofen and diacerein in combination may cause some, particularly gastrointestinal, side effects. Flurbiprofen shows burning sensation in the gastrointestinal system and diacerein shows gastrointestinal side effect of diarrhea as well. Moreover, the use of diacerien with flurbiprofen can cause additional effects. Avoiding these systemic adverse effects of flurbiprofen and diacerein is very important for the patient.

Therefore, in prior art, flurbiprofen and diacerein has been used in combination. However, none of them comprises controlled release flurbiprofen and diacerein in the same oral dosage form.

In this invention, a controlled release layer has been specifically formulated as to contain flurbiprofen and release slowly it over in a desired period of time, until the next dosage is administered. The formulation of this present invention also comprises an immediate release layer comprising diacerein which has a different mechanism of action in the treatment of osteoarthritis. As a result, an effective and safe administration of flurbiprofen and diacerein combination has been achieved for the patients' compliance.

### Background of the Invention

The main embodiment of this present invention is a pharmaceutical combination comprising: (1) a controlled release layer comprising flurbiprofen or a pharmaceutically acceptable salt thereof, (2) an immediate release layer comprising diacerein or a pharmaceutically acceptable salt thereof, and pharmaceutically acceptable excipients.

In one embodiment, the pharmaceutical combination is in the form of multilayer tablet. In order to combine two different molecules with two different release profile in one dosage form, in this invention two layers that have different release profiles have been designed; a controlled release layer, an immediate release layer and optionally an intermediate layer.

In one embodiment, flurbiprofen or a pharmaceutically acceptable salt thereof is present in an amount of between 20-80 % by weight of controlled release layer and the diacerein or a pharmaceutically acceptable salt thereof is present in an amount of between 5 - 90% by weight of immediate release layer.

In one embodiment, the ratio of flurbiprofen to diacerein is between 10:1 to 1:1 (w/w), preferably 6:1 to 1:1 (w/w) and more preferably 6:1 to 4:1 (w/w). To achieve different desired dose of active agents, these ratios have been used in separate layers.

In one embodiment, the pharmaceutical combination further comprises a release rate controlling agent in controlled release layer comprising flurbiprofen. As used herein, the term "release rate controlling agent" is defined as the pharmaceutical ingredient that provides controlled release, modified release, retarded release, slow release, sustained release, prolonged release, zero point order release or extended release.

The term "controlled release" is defined as reaching desired plasma levels of an active agent of interest throughout a determined period of time, and providing the drug release at a uniform and constant rate. In this present invention, the aim of using release rate controlling agent was to improve dissolution characteristics while achieving a desired controlled release flurbiprofen profile.

In one embodiment, release rate controlling agent has been used in a specific ratio and it has been surprisingly found that controlled release of flurbiprofen has been achieved without affecting diacerein release profile in immediate release layer. In this the pharmaceutical combination of this present invention, the ratio of flurbiprofen to release rate controlling agent is between 1:2 to 16:1 (w/w), preferably 1:2 to 10:1 (w/w), more preferably it is 1:2 to 5:1 (w/w) in controlled release layer.

According to this embodiment, release rate controlling agent is selected from the group comprising polyvinyl acetate, polyvinylpyrrolidone,HPMC (hydroxyl propyl methylcellulose), hydroxyethyl methylcellulose, hydroxypropyl cellulose, hydroxypropylethylcellulose, ethylcellulose, methacrylicacid - ethyl acrylate copolymer, polymethylmetacrylate or copolymers, polyvinyl alcohol, glycerylbehenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers, lambda (λ) carrageenan, ethylcellulosepolymers or copolymers, polymethacrylates or copolymers, natural gums such as xanthan gum, surfactants as cetostearyl alcohol, cethyl alcohol or mixtures thereof. Preferably, it is the mixture of polyvinyl acetate and polyvinyl pyrrolidone.

In this invention, the mixture of polyvinyl acetate and polyvinyl pyrrolidone comprised of 80 % of polyvinyl acetate and 19 % of polyvinyl pyrrolidone by weight of total mixture.

It is difficult to develop a tablet formulation comprising flurbiprofen has low solubility in terms of the process. Herein, the mixture of polyvinyl acetate and polyvinyl pyrrolidone is used to further improve the process conditions by increasing the flurbiprofen solubility, as well as being controlled release agent.

According to this embodiment, the mixture of polyvinyl acetate and polyvinyl pyrrolidone has been used in a specific ratio and it has been found that controlled release of flurbiprofen has been achieved without affecting diacerein release profile in immediate release layer. In this pharmaceutical combination of this present invention, the ratio of flurbiprofen to the mixture of polyvinyl acetate and polyvinyl pyrrolidone is between 1:2 to 16:1 (w/w), preferably 1:2 to 10:1 (w/w), more preferably it is 1:2 to 5:1 (w/w) in controlled release layer.

According to this embodiment, binder is selected from the group comprising starch, polyvinylpyrrolidone (povidone), pregelatinized starch, lactose, polymethacrylate, hydroxypropyl methyl cellulose (HPMC), hydroxypropyl cellulose (HPC), carboxymethyl cellulose (CMC), methyl cellulose (MC), hydroxyethyl cellulose, sodium carboxy methyl cellulose (NaCMC), carboxymethyl cellulose calcium, ethyl cellulose, polyethylene oxide, gelatin, xanthan gum, guar gum, alginate, carrageenan, pectin, carbomer, cellulose acetate phthalate, hydroxy propyl starch, polaxomer, polyethylene glycol and mixtures thereof. Preferably, it is starch.

In one embodiment, the pharmaceutical combination further comprises starch.

According to one embodiment, due to starch used as a binder in the formulation, hardness of the tablet has been improved, layers adhere to each other and robust multilayer tablet with desired dissolution has been achieved.

In one embodiment, the pharmaceutical combination is in the form of trilayer tablet.

In one embodiment, the pharmaceutical combination is in the form of bilayer tablet.

In one embodiment, the pharmaceutical combination is in the form of coated tablet.

In one embodiment, the pharmaceutical combination is in the form of tablet in tablet.

In one embodiment, the pharmaceutical combination is in the form of inlay tablet.

Coating material may be selected from the group comprising methylacrylate copolymer (Eudragit EPO) and other polymethylmetacrylate copolymers (Eudragit), polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA), and all kinds of OpadryTM such as Opadry pink II, as well as pigments, dyes, titanium dioxide and iron oxide.

Suitable diluents may include but not limited to microcrystalline cellulose, lactose, corn starch, modified corn starch, calcium phosphate, sugar, dextrose, mannitol, sorbitol, starch, pregelatinized starch and mixtures thereof.

Suitable disintegrants may include but not limited to microcrystalline cellulose, croscarmellose sodium, xylitol, polyplasdone (1-ethenylpyrrolidin-2-one), crospovidone, hydroxypropyl cellulose, low-substituted hydroxypropyl cellulose (L-HPC) and sodium starch glycolate or mixtures thereof.

Suitable glidants may include but not limited to colloidal silicon dioxide, silicon dioxide, talc, magnesium trisilicate, starch or silicon hydrogel or mixtures thereof.

Lubricants according to the present invention include, but not limited to magnesium stearate, sodium stearyl fumarate, polyethylene glycol, stearic acid, metal stearates, boric acid, sodium chloride benzoate and acetate, sodium or magnesium lauryl sulfate or mixtures thereof.

Suitable plasticizers may include but not limited to triethyl citrate, triacetin, citric acid esters, phthalic acid esters, dibutylsebacate, cetyl alcohol, polyethylene glycol, polysorbate or mixtures thereof.

Suitable surfactants may include but not limited to dioctylsulfosuccinate, polysorbates and polyoxyethylene alkyl esters and ethers thereof, glycerylmonolauratesaponins, sorbitanlaurate, sodium lauryl sulfate, magnesium lauryl sulfate or mixtures thereof.

Suitable preservatives may include but not limited to methyl paraben, propyl paraben and salts thereof (e.g. sodium or potassium salts), sodium benzoate, citric acid, benzoic acid, butylatedhydroxytoluene and butylatedhydroxyanisole or mixtures thereof.

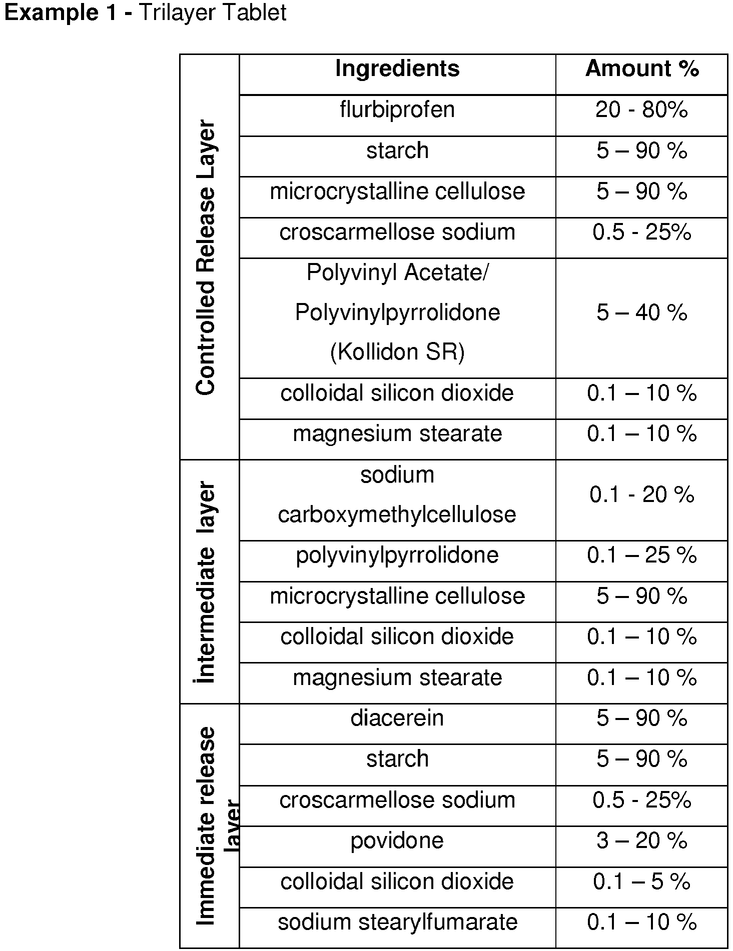

The process of the formulation is carried out as follows:
**Controlled release layer**:Flurbiprofen, a portion of microcrystalline cellulose, starch, croscarmellose sodium and polyvinyl acetate/ polyvinylpyrrolidoneare mixed; colloidal silicon dioxide and rest of the microcrystalline cellulose are sieved, added into the mixture obtained and mixed. Then,tablets are compressed into bricks, crushed and sieved. Magnesium stearate is added into the mixture obtained and mixed.

**Immediate release layer:** Diacerein, a portion of starch, croscarmellose sodium and povidone are mixed. Colloidal silicon dioxide and rest of the starch are sieved, added into the mixture obtained and mixed. Then, Sodium stearylfumarate is added into this mixture and mixed.

**Intermediate layer**:Sodium carboxymethyl cellulose, polyvinylpyrrolidone and a portion of microcrystalline cellulose are mixed. Rest of the microcrystalline cellulose and colloidal silicon dioxide are sieved and added into the mixture obtained and mixed. Then, magnesium stearate is added into the mixture obtained and mixed.

Homogeneous mixtures obtained for each layer are compressed so as to obtain a trilayer tablet with the barrier layer provided in the middle thereof.

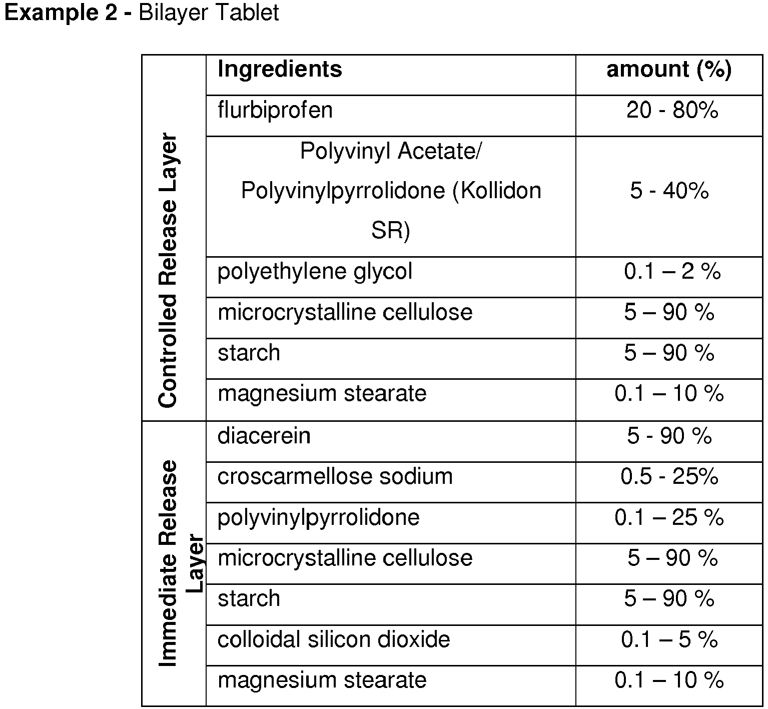

The process of the formulation is carried out as follows:
**Controlled release layer:** polyethylene glycol is dissolved in water and half of polyvinyl acetate/ polyvinylpyrrolidone is added and mixed together (mixturei). Flurbiprofen and microcrystalline cellulose is added to high-shear mixture, then mixture i is added to this mixture and the mixture is granulated while the mixer is open. Then, granules are dried in fluid bed dryer and sieved. The sieved granules obtained are coated with the rest of the polyvinyl acetate/ polyvinylpyrrolidonein fluid bed dryer, finally starch and magnesium stearate are added to this granules as an external phase.

**Immediate release layer:** diacerein,microcrystalline cellulose, starch and half of the croscarmellose sodium are mixed together, then they are granulated with the water-solution of polyvinylpyrrolidone. Obtained granules are then sieved and dried in oven. The dried granules are also sieved and colloidal silicon dioxide, magnesium stearate and rest of the croscarmellose sodium are added as an external phase.

Finally, compression step is performed to form the bilayer tablets

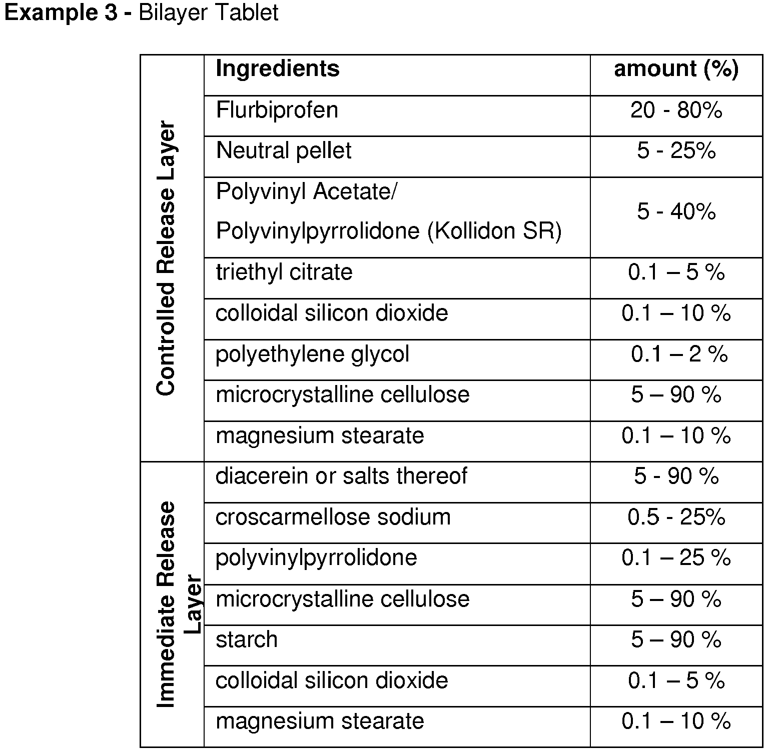

The process of the formulation is carried out as follows:
**Controlled release layer:** Water is added to a tank which comprise a mechanic stirrer and a homogenizer (mixture i), and triethly citrate is added to this tank and it is mixed with the mechanic stirrer (mixture ii). Flurbiprofen is added to mixture ii while the homogenizer is open to obtain the mixture iii. Polyvinyl acetate/ polyvinylpyrrolidoneis added to mixture III while the homogenizer is closed and it is mixed until a homogenous mixture is obtained and no bubles remain (mixture iv). Then, neutral pellets are added to fluid bed dryer and the mixture iv is coated on the pellets with spraying. After the coating step, colloidal silicon dioxide is added on the coated pellets and coated pellets are sieved. Sieving is important in this step to prevent the coated pellets from sticking to each other. Finally, polyethylene glycol, microcrystalline cellulose and magnesium stearate are added to these granules as an external layer.

**Immediate release layer:** diacerein,microcrystalline cellulose, starch and half of the croscarmellose sodium are mixed together, then they are granulated with the water-solution of polyvinylpyrrolidone. Obtained granules are then sieved and dried in oven. Dried granules are also sieved and colloidal silicon dioxide, magnesium stearate and rest of the croscarmellose sodium are added as an external layer.

Finally, compression step is performed to form the bilayer tablets.

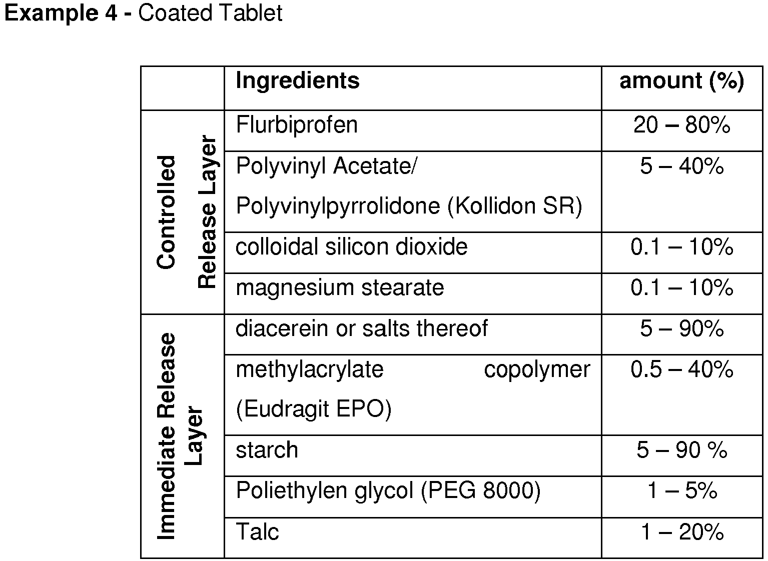

The process of the formulation is carried out as follows:
**Controlled release layer**:Flurbiprofen, polyvinyl Acetate/ polyvinylpyrrolidone , colloidal silicon dioxide and magnesium stearate are mixed and granulated with high shear granulator or they are obtained from extruder to become pellets by spheronizer and after dried by fluid bed drier or dried in oven, they are sieved and pressed or matrix tabletsare pressed by direct compression.

**Immediate release layer:** Water is used for the preparation of film coating mixture.Diacereinand Eudragit EPO are dissolved in water and PEG, starch and then talc is added to this mixture and it is mixed until having a homogenous mixture.The pressed controlled release flurbiprofen tablets are coated with this coating suspension.These coated tablets, preferably are coated by a coating material including conventional coating polymers like Opadry^{®}.

## Claims

1. A pharmaceutical combination comprising: (1) a controlled release layer comprising flurbiprofen or a pharmaceutically acceptable salt thereof and (2) an immediate release layer comprising diacerein or a pharmaceutically acceptable salt thereof, and pharmaceutically acceptable excipients.

2. The pharmaceutical combination according to claim 1, wherein the flurbiprofen or a pharmaceutically acceptable salt thereof is present in an amount of between 20-80 % by weight of controlled release layer and the diacerein or a pharmaceutically acceptable salt thereof is present in an amount of between 5 - 90% by weight of immediate release layer.

3. The pharmaceutical combination according to claim 2, wherein the ratio of flurbiprofen to diacerein is between 10:1 to 1:1 (w/w), preferably 6:1 to 1:1 (w/w) and more preferably 6:1 to 4:1 (w/w)

4. The pharmaceutical combination according to any preceding claims, further comprising a release rate controlling agent.

5. The pharmaceutical combination according to any preceding claims, wherein the ratio of flurbiprofen to release rate controlling agent is between 1:2 to 16:1 (w/w), preferably 1:2 to 10:1 (w/w), more preferably it is 1:2 to 5:1 (w/w) in controlled release layer.

6. The pharmaceutical combination according to any preceding claims, wherein the release rate controlling agent is selected from the group comprising polyvinyl acetate, polyvinylpyrrolidone, HPMC (hydroxyl propyl methylcellulose), hydroxyethyl methylcellulose, hydroxypropyl cellulose, hydroxypropylethylcellulose, ethylcellulose, methacrylic acid - ethyl acrylate copolymer, polymethylmetacrylate or copolymers, polyvinyl alcohol, glycerylbehenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene - alkyl ethers, lambda (λ) carrageenan, ethylcellulose polymers or copolymers, polymethacrylates or copolymers, natural gums such as xanthan gum, surfactants as cetostearyl alcohol, cethyl alcohol or mixtures thereof. Preferably, it is the mixture of polyvinyl acetate and polyvinyl pyrrolidone.

7. The pharmaceutical combination according to any preceding claims, wherein the ratio of flurbiprofen to the mixture of polyvinyl acetate and polyvinyl pyrrolidone is between 1:2 to 16:1 (w/w), preferably 1:2 to 10:1 (w/w), more preferably it is 1:2 to 5:1 (w/w) in controlled release layer.

8. The pharmaceutical combination according to any preceding claims, wherein the formulation further comprising starch.

9. The pharmaceutical combination according to any preceding claims, the pharmaceutical combination is in the form of multilayer tablet.

10. The pharmaceutical combination according to any preceding claims, wherein the formulation is in the form of trilayer tablet.

11. The pharmaceutical combination according to any preceding claims, wherein the formulation is in the form of bilayer tablet.

12. The pharmaceutical combination according to any preceding claims, wherein the formulation is in the form of coated tablet.

13. The pharmaceutical combination according to any preceding claims, wherein the formulation is in the form of tablet in tablet.

14. The pharmaceutical combination according to any preceding claims, wherein the formulation is in the form of inlay tablet.

## Patentansprüche

1. Pharmazeutische Kombination aufweisend: (1) eine kontrollierte-Abgabe-Schicht aufweisend Flurbiprofen oder ein pharmazeutisch akzeptables Salz hiervon und (2) eine direkte-Abgabe-Schicht aufweisend Diacerein oder ein pharmazeutisch akzeptables Salz hiervon und pharmazeutisch akzeptable Hilfsstoffe.

2. Pharmazeutische Kombination nach Anspruch 1,
wobei das Flurbiprofen oder ein pharmazeutisch akzeptables Salz hiervon in einer Menge von zwischen 20 - 80 Gew.% der kontrollierten-Abgabe-Schicht vorhanden ist und das Diacerein oder ein pharmazeutisch akzeptables Salz hiervon in einer Menge von zwischen 5 - 90 Gew.% der direkten-Abgabe-Schicht vorhanden ist.

3. Pharmazeutische Kombination nach Anspruch 2,
wobei das Verhältnis von Flurbiprofen zu Diacerein zwischen 10:1 bis 1:1 (w/w), vorzugsweise 6:1 bis 1:1 (w/w) und noch bevorzugterer Weise 6:1 bis 4:1 (w/w) ist.

4. Pharmazeutische Kombination nach einem der voranstehenden Ansprüche,
weiter aufweisend ein freisetzungsratenkontrollierendes Mittel.

5. Pharmazeutische Kombination nach einem der voranstehenden Ansprüche, wobei das Verhältnis von Flurbiprofen zu dem freisetzungsratenkontrollierenden Mittel zwischen 1:2 bis 16:2 (w/w), vorzugsweise 1:2 bis 10:1 (w/w), noch bevorzugterer Weise 1:2 bis 5:1 (w/w) in der kontrollierten-Abgabe-Schicht vorliegt.

6. Pharmazeutische Kombination nach einem der voranstehenden Ansprüche, wobei das freisetzungsratenkontrollierende Mittel aus der Gruppe aufweisend Polyvinylacetat, Polyvinylpyrrolidon, HPMC (Hydroxyl Propyl Methylcellulose), Hydroxyethyl Methylcellulose, Hydroxypropyl Cellulose, Hydroxypropylethylcellulose, Ethylcellulose, Methacrylsäure - Ethyl Acrylat Copolymer, Polymethylmetacrylat oder Copolymere, Polyvinyl Alkohol, Glycerylbehenat, Polyethylen Oxid, Polyethylen Glycol, Cellulose Acetat, Vinyl Acetat/Crotonsäure Copolymere, Maleinsäureanhydrid/Methyl Vinyl Ether Copolymere, Copolymere von Acrylischen oder Methacrylsäure Estern, Polyoxyethylene - Alkyl Ethers, Lambda (A) Carrageen, Ethylcellulose Polymere oder Copolymere, Polymethacrylate oder Copolymere, natürliche Gummis wie Xanthan Gummi, grenzflächenaktive Substanzen wie Cetostearylalkohol, Cethyl Alkohol oder eine Mischung davon ausgewählt ist. Bevorzugter Weise ist es die Mischung von Polyvinyl Acetat und Polyvinyl Pyrrolidon.

7. Pharmazeutische Kombination nach einem der voranstehenden Ansprüche,
wobei das Verhältnis von Flurbiprofen zu der Mischung von Polyvinyl Acetat und Polyvinyl Pyrrolidon zwischen 1:2 bis 16:1 (w/w), vorzugsweise 1:2 bis 10:1 (w/w), noch bevorzugterer Weise 1:2 bis 5:1 (w/w) in der kontrollierten-Abgabe-Schicht ist.

8. Pharmazeutische Kombination nach einem der voranstehenden Ansprüche,
wobei die Zusammensetzung weiter Stärke aufweist.

9. Pharmazeutische Kombination nach einem der voranstehenden Ansprüche,
wobei die pharmazeutische Zusammensetzung in der Form einer mehrschichtigen Tablette ist.

10. Pharmazeutische Kombination nach einem der voranstehenden Ansprüche,
wobei die Zusammensetzung in der Form einer dreischichtigen Tablette ist.

11. Pharmazeutische Kombination nach einem der voranstehenden Ansprüche,
wobei die Zusammensetzung in der Form einer zweischichtigen Tablette ist.

12. Pharmazeutische Kombination nach einem der voranstehenden Ansprüche,
wobei die Zusammensetzung in der Form einer beschichteten Tablette ist.

13. Pharmazeutische Kombination nach einem der voranstehenden Ansprüche,
wobei die Zusammensetzung in der Form einer Tablette in einer Tablette ist.

14. Pharmazeutische Kombination nach einem der voranstehenden Ansprüche,
wobei die Zusammensetzung in Form einer Einlagetablette ist.

## Revendications

1. Combinaison pharmaceutique comprenant : (1) une couche à libération contrôlée comprenant du flurbiprofène ou un sel pharmaceutiquement acceptable de celui-ci et (2) une couche à libération immédiate comprenant de la diacéréine ou un sel pharmaceutiquement acceptable de celle-ci, et des excipients pharmaceutiquement acceptables.

2. Combinaison pharmaceutique selon la revendication 1, dans laquelle le flurbiprofène ou un sel pharmaceutiquement acceptable de celui-ci est présent en une quantité comprise entre 20 et 80 % en poids de couche à libération contrôlée et la diacéréine ou un sel pharmaceutiquement acceptable de celle-ci est présent(e) en une quantité comprise entre 5 et 90 % en poids de couche à libération immédiate.

3. Combinaison pharmaceutique selon la revendication 2, dans laquelle le rapport du flurbiprofène à la diacéréine est compris entre 10:1 et 1:1 (pds/pds), de préférence entre 6:1 et 1:1 (pds/pds) et plus préférentiellement entre 6:1 et 4:1 (pds/pds).

4. Combinaison pharmaceutique selon l'une quelconque des revendications précédentes, comprenant en outre un agent de contrôle du taux de libération.

5. Combinaison pharmaceutique selon l'une quelconque des revendications précédentes, dans laquelle le rapport du flurbiprofène à l'agent de contrôle du taux de libération est compris entre 1:2 et 16:1 (pds/pds), de préférence entre 1:2 et 10:1 (pds/pds), plus préférentiellement entre 1:2 et 5:1 (pds/pds) dans la couche de libération immédiate.

6. Combinaison pharmaceutique selon l'une quelconque des revendications précédentes, dans laquelle l'agent de contrôle du taux de libération est sélectionné parmi le groupe comprenant de l'acétate de polyvinyle, de la polyvinylpyrrolidone, de l'HPMC (hydroxylpropylméthylcellulose), de l'hydroxyéthylméthylcellulose, de l'hydroxypropylcellulose, de l'hydroxypropyléthylcellulose, de l'éthylcellulose, d'un copolymère d'acide méthacrylique - acrylate d'éthyle, de polymétacrylate de méthyle ou copolymères, d'alcool polyvinylique, de glycérylbéhénate, d'oxyde de polyéthylène, de polyéthylène glycol, d'acétate de cellulose, de copolymères d'acétate de vinyle/acide crotonique, de copolymères d'anhydride maléique/éther méthylique de vinyle, de copolymère d'esters d'acide acrylique ou méthacrylique, d'éthers de polyoxyéthylène-alkyle, de carraghénane lambda-(λ), de polymères ou copolymères d'éthylcellulose, de polyméthacrylates ou copolymères, de gommes naturelles comme la gomme xanthane, de tensioactifs comme l'alcool cétostéarylique, l'alcool cétylique ou des mélanges de ceux-ci. De préférence, il s'agit du mélange d'acétate de polyvinyle et de polyvinylpyrrolidone.

7. Combinaison pharmaceutique selon l'une quelconque des revendications précédentes, dans laquelle le rapport du flurbiprofène au mélange d'acétate de polyvinyle et de polyvinylpyrrolidone est compris entre 1:2 et 16:1 (pds/pds), de préférence 1:2 et 10:1 (pds/pds), plus préférentiellement entre 1:2 et 5:1 (pds/pds) en couche de libération contrôlée.

8. Combinaison pharmaceutique selon l'une quelconque des revendications précédentes, dans laquelle la formulation comprend en outre de l'amidon.

9. Combinaison pharmaceutique selon l'une quelconque des revendications précédentes, la combinaison pharmaceutique étant sous forme de comprimé multicouche.

10. Combinaison pharmaceutique selon l'une quelconque des revendications précédentes, dans laquelle la formulation est sous forme de comprimé tricouche.

11. Combinaison pharmaceutique selon l'une quelconque des revendications précédentes, dans laquelle la formulation est sous forme de comprimé bicouche.

12. Combinaison pharmaceutique selon l'une quelconque des revendications précédentes, dans laquelle la formulation est sous forme de comprimé enrobé.

13. Combinaison pharmaceutique selon l'une quelconque des revendications précédentes, dans laquelle la formulation est sous forme de comprimé-dans-comprimé.

14. Combinaison pharmaceutique selon l'une quelconque des revendications précédentes, dans laquelle la formulation est sous forme de comprimé incrusté.
